# EUROPEAN PATENT APPLICATION

(11) **EP 4 215 602 A1**
(43) Date of publication of application: **26.07.2023**
(21) Application number: 21880190.0
(22) Date of filing: 14.10.2021
(51) Int. Cl.: C12N 5/0775, C12N 5/071, C12N 5/077

(54) **METHOD FOR PRODUCING CARDIAC MUSCLE STEM/PRECURSOR CELLS AND METHOD FOR INHIBITING MYOCARDIAL FIBROSIS**

(30) Priority: 14.10.2020 JP 2020173581
(71) Applicant: Da Vinci Universale Co., Ltd., Tokyo, 100-0005 (JP)
(72) Inventor: MURANAKA, Asao, Kashiwa-Shi, Chiba 277-0832 (JP); OCHIYA, Takahiro, Tokyo 104-0053 (JP); PRIETO-VILA, Marta, Tokyo 160-0023 (JP)
(74) Representative: Barker Brettell LLP
(86) International application number: PCT/JP2021/038071
(87) International publication number: WO 2022/080455

(57) **Abstract**

Provided is a method for efficiently reprogramming mature and juvenile cardiomyocytes into myocardial stem/progenitor cells and/or suppressing fibrosis, without genetic modification.

Myocardial stem/progenitor cells can be maintained by culturing primary cardiomyocytes in the presence of a ROCK inhibitor. Furthermore, fibrosis is suppressed by allowing cardiomyocytes cultured in the presence of these low molecular weight compounds or an exosome or secretome derived from the cardiomyocytes to act on cardiac fibroblasts.

## Description

### CROSS-REFERENCE TO RELATED APPLICATION

The present international application claims priority based on Japanese Patent Application No. 2020-173581 filed with the Japan Patent Office on October 14, 2020, and the entire contents of Japanese Patent Application No. 2020-173581 are incorporated herein by reference.

### TECHNICAL FIELD

The present invention relates to a method for producing a myocardial stem/progenitor cell using a low molecular weight compound, a method for suppressing fibrosis of cardiomyocytes, or a long-term culturing method.

### BACKGROUND ART

Remarkable progress of stem cell biology occupies an important position for its application in myocardial regenerative medicine, but has not yet been realized. Induced pluripotent stem cells (iPS cells), which are one of the most expected cell sources, still have a risk of tumor formation, and it is difficult to put them into practical use for real clinical applications, although clinical studies have been conducted (Non Patent Documents 1 to 3). On the other hand, recent studies have shown that cells of different lineages can be directly converted (direct reprogramming) into myocardial progenitor cell-like cells. However, as in the case of iPS cells, the direct reprogramming involves genetic modification by introduction of genes such as Gata4, Mef2c, and Tbx5, and thus still has an unexpected risk and cannot be applied to regenerative medicine (Non-Patent Document 4).

Recently, the fact that cardiac fibroblasts are reprogrammed into cardiomyocytes has been successively reported (Non-Patent Document 5). These innovative findings give great insight not only in myocardial stem cell theory but also in myocardial regeneration studies. That is, if such reprogramming can be reproduced, the myocardial stem/progenitor cells thus obtained are expected to be innovative cell sources in myocardial regenerative medicine. However, there is no known method of reprogramming cardiomyocytes at various stages into myocardial stem/progenitor cells without genetic modification.

The present inventors and other groups have previously reported that a combination of certain small molecule inhibitors contributes to induction and maintenance of pluripotency of stem cells in the liver, stomach, and the like (Patent Document 1 and Non-Patent Documents 6 to 9). However, the relationship with reprogramming from mature and juvenile cardiomyocytes into myocardial stem/progenitor cells for small molecule inhibitors has not been reported so far.

### PRIOR ART DOCUMENTS

### PATENT DOCUMENTS

Patent Document 1: JP 2018-079714 A1

### NON-PATENT DOCUMENTS

Non-Patent Document 1: Cell. 2014 Oct 9;159(2):428-39
Non-Patent Document 2: Cell Metab. 2016 Apr 12; 23(4): 622-634
Non-Patent Document 3: IntechOpen, 2019DOI: http://dx.doi.org/10.5772/intechopen.88878.
Non-Patent Document 4: Stem Cells International, Volume 2018, Article ID 1435746, https://doi.org/10.1155/2018/1435746
Non-Patent Document 5: Circulation Report. 2019, review, doi:10.1253/circrep.CR-19-0104
Non-Patent Document 6: Proc Natl Acad Sci U S A. 2010 Aug 10; 107(32): 14223-8.
Non-Patent Document 7: Cell Stem Cell. 2017 Jan 5;20(1):41-55
Non-Patent Document 8: Cell Stem Cell. 2016 Oct 6;19(4):449-461
Non-Patent Document 9: eLIFE, 2019, https://doi.org/10.7554/eLife.47313.001

### SUMMARY OF THE INVENTION

### PROBLEMS TO BE SOLVED BY THE INVENTION

An object of the present invention is to provide a method for efficiently reprogramming mature and juvenile cardiomyocytes into myocardial stem/progenitor cells without genetic modification. In another aspect, an object of the present invention is to provide a method for maintaining myocardial stem/progenitor cells in a non-differentiated state for a long term. In yet another aspect, an object of the present invention is to provide a method for suppressing fibrosis of fibroblasts and/or defiberizing fibrotic fibroblasts. In still yet another aspect, an object of the present invention is to provide a method for suppressing onset and/or exacerbation of a cardiovascular disease and activating development and/or functions of a cardiovascular system.

### MEANS FOR SOLVING THE PROBLEMS

In order to achieve the above object, the present inventors have repeatedly studied a low molecular weight compound that can contribute to reprogramming of cardiomyocytes, and, as a result, have found that, when cardiomyocytes are cultured in the presence of a Rho kinase inhibitor, expression of an undifferentiation marker is maintained, and that an increase in expression of a mature cardiomyocyte marker, a senescence marker, and/or an endothelial cell marker can be suppressed. This has led to the successful reprogramming of cardiomyocytes with a Rho kinase inhibitor as a low molecular weight compound. In addition, from the fact that the cardiomyocytes cultured in the presence of the Rho kinase inhibitor can proliferate for a long term while maintaining the state as stem cells, the present inventors have found that the Rho kinase inhibitor brings about maintenance of an undifferentiated state. In addition, the present inventors have found that, in cardiomyocytes treated with a ROCK inhibitor of the present invention, signaling pathways related to cardiovascular diseases are suppressed, and that signaling pathways associated with cardiovascular development and/or functions are activated. Furthermore, the present inventors have found that co-culture of cardiomyocytes and fibroblasts cultured in the presence of these low molecular weight compounds and culture of fibroblasts in the presence of exosomes derived from the cardiomyocytes can suppress fibrosis. In addition, the present inventors have found that the exosomes are capable of defiberizing fibrotic cells.

### EFFECTS OF THE INVENTION

According to the present invention, myocardial stem/progenitor cells having self-proliferation capability can be safely and quickly induced from various cardiomyocytes without genetic modification. In addition, according to the present invention, cardiomyocytes including myocardial stem/progenitor cells can be stably cultured for a long term. In addition, treatment of cardiomyocytes with the ROCK inhibitor of the present invention can be expected to suppress the onset and/or exacerbation of a cardiovascular disease and to activate the development and/or functions of the cardiovascular system. In addition, cardiomyocytes treated with the ROCK inhibitor of the present invention or a secretome or exosome derived from the cardiomyocytes can safely and quickly suppress fibrosis of fibroblasts and/or defiberize fibrotic fibroblasts. The method of the present invention enables supply of cardiomyocytes in autologous/allogenic transplantation, and can also be used for treatment and prevention of cardiomyopathy, myocarditis, and other diseases associated with fibrosis of a cardiac muscle, and can also be used for production of model cells for these diseases, evaluation of therapeutic drugs, evaluation of cardiac toxicity, and the like. Furthermore, the method of the present invention can safely and quickly induce and/or maintain myocardial stem/progenitor cells from cardiomyocytes without genetic modification, and thus can be applied to cardiac function regenerative medicine.

### BRIEF DESCRIPTION OF THE DRAWINGS

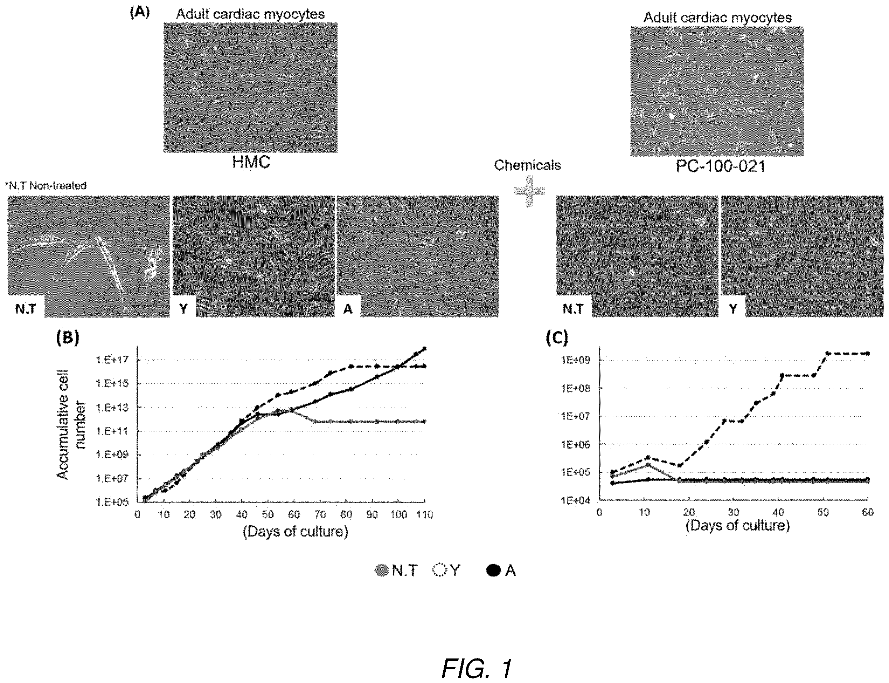

FIG. 1 shows changes in cell morphology and proliferation of cardiomyocytes treated with a TGFβ receptor inhibitor or a ROCK inhibitor. A: Photographs showing that cardiomyocytes take the form of myocardial stem/progenitor cells via long-term culture under treatment with the TGFβ receptor inhibitor (A) alone and treatment with the ROCK inhibitor (Y) alone. B: A graph showing proliferation of HCM cells under conditions of administration of the TGFβ receptor inhibitor (A) alone and administration of the ROCK inhibitor (Y) alone. C: A graph showing proliferation of primary human coronary artery smooth muscle cells PC-100-021 cells under the conditions of administration of the TGFβ receptor inhibitor (A) alone and administration of the ROCK inhibitor (Y) alone.

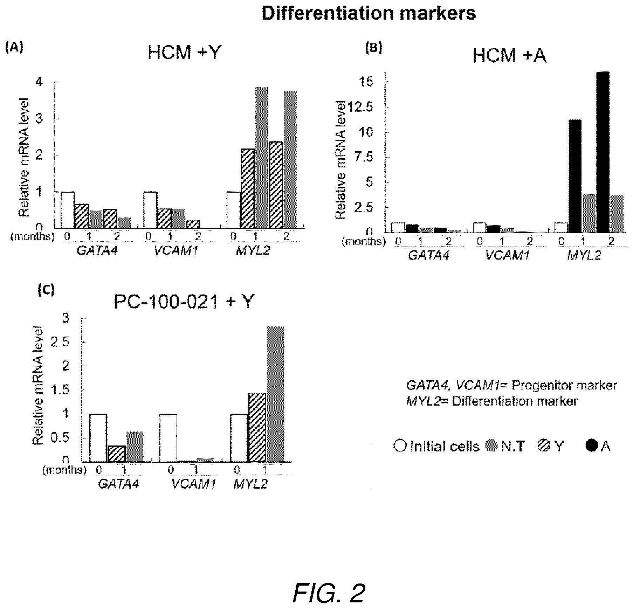

FIG. 2 includes graphs showing mRNA expression of myocardial progenitor cell markers (GATA4 and VCAM-1) and MYL2 as a maturation marker for cardiomyocytes, in the cardiomyocytes treated with the TGFβ receptor inhibitor (A) or the ROCK inhibitor (Y). The vertical axis represents an expression amount (Relative mRNA level) of each mRNA when an expression amount of the cells before culture is 1, the letters on the horizontal axis represent target mRNAs, and the numerical values represent culture periods (month). In each month for each mRNA, a bar graph on the left shows data on an agent-treated group and a bar graph on the right shows data on an agent-untreated group (control).

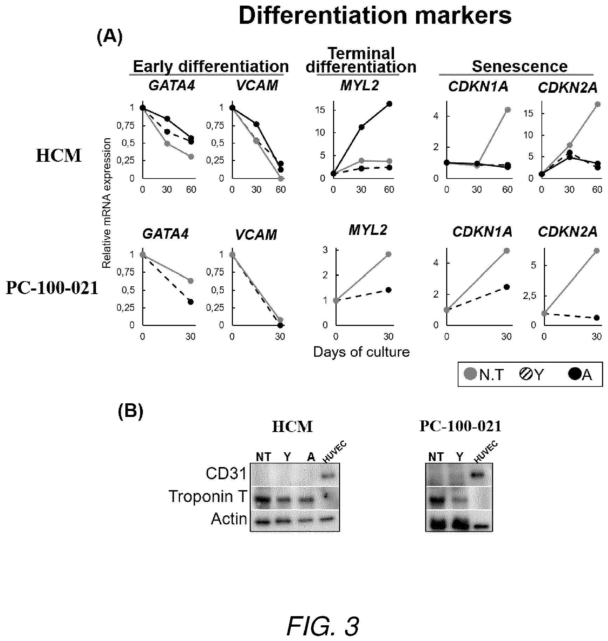

FIG. 3 A: Graphs showing mRNA expression of the myocardial progenitor cell markers (GATA4 and VCAM-1), the maturation marker (MYL2), and senescence markers (CDKN1A and CDKN2A) for cardiomyocytes, in the cardiomyocytes treated with the TGFβ receptor inhibitor (A) or the ROCK inhibitor (Y). In each graph, the vertical axis represents the expression amount (Relative mRNA expression) of each mRNA when the expression amount of the cells before culture is 1, and the numerical values on the horizontal axis represent culture periods (day). B: Photographs showing the results of confirming, by Western blot, the expression of an endothelial cell marker (CD31), a muscle cell marker (Troponin T), and Actin, in primary human cardiomyocytes (HCM) or primary human coronary artery smooth muscle cells (PC-100-021) treated with the TGFβ receptor inhibitor (A) or the ROCK inhibitor (Y). NT indicates an agent-untreated control, and HUVEC indicates a result of using human umbilical vein endothelial cells (CD31-positive as the endothelial cell marker).

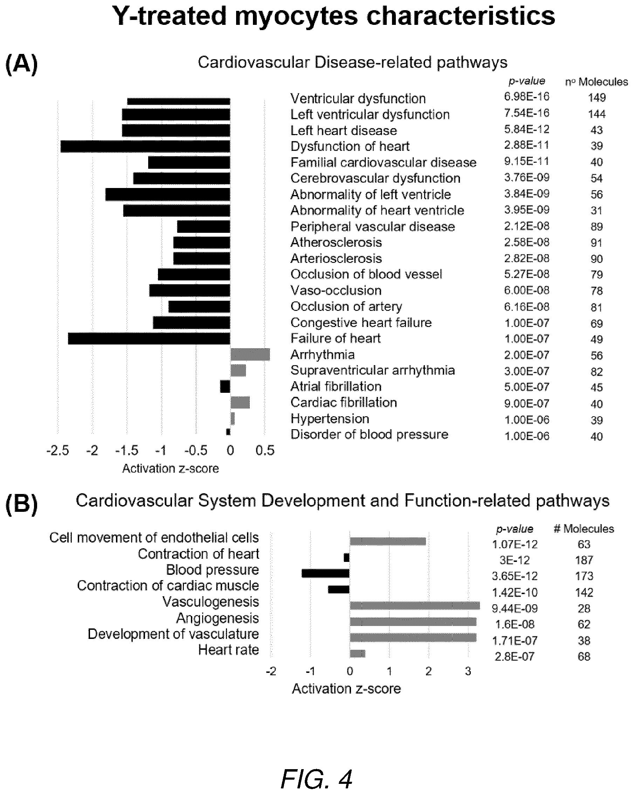

FIG. 4 includes graphs showing the results of estimating a signaling pathway change by comparing Total RNA of cardiomyocytes treated with the ROCK inhibitor with Total RNA of untreated cardiomyocytes using Ingenuity (registered trademark) Pathway Analysis (IPA). A: Estimation results of changes in signaling pathways associated with cardiovascular disease. B: Estimation results of changes in signaling pathways related to development and functions of the cardiovascular system. As for Activation z-score in the graph, see Bioinformatics. (2014); See 30 (4): 523-530. # Molecules represents the number of molecules.

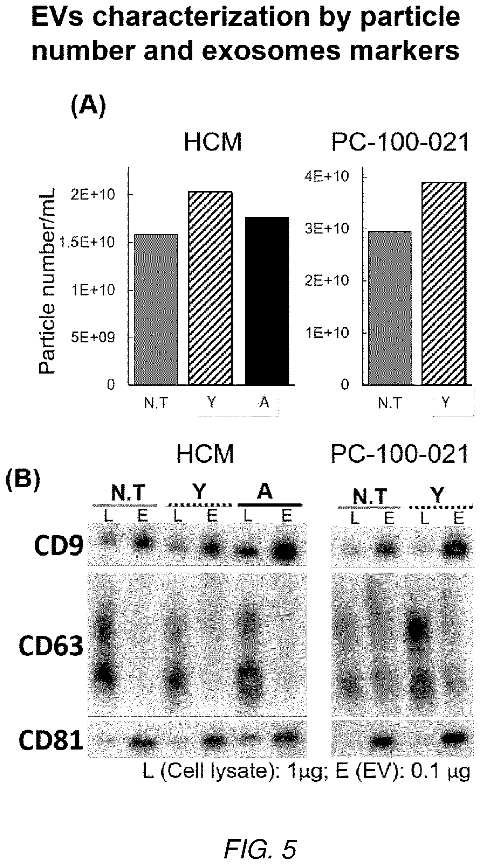

FIG. 5 A: Graphs showing the number of particles of extracellular vesicles purified by ultracentrifugation from a culture supernatant of cardiomyocytes treated with the TGFβ receptor inhibitor or the ROCK inhibitor. B: Photographs showing the results of identifying, by Western blotting, a CD9 molecule, a CD63 molecule, and a CD81 molecule in the extracellular vesicles purified by ultracentrifugation from the culture supernatant of the cardiomyocytes treated with the TGFβ receptor inhibitor and the ROCK inhibitor.

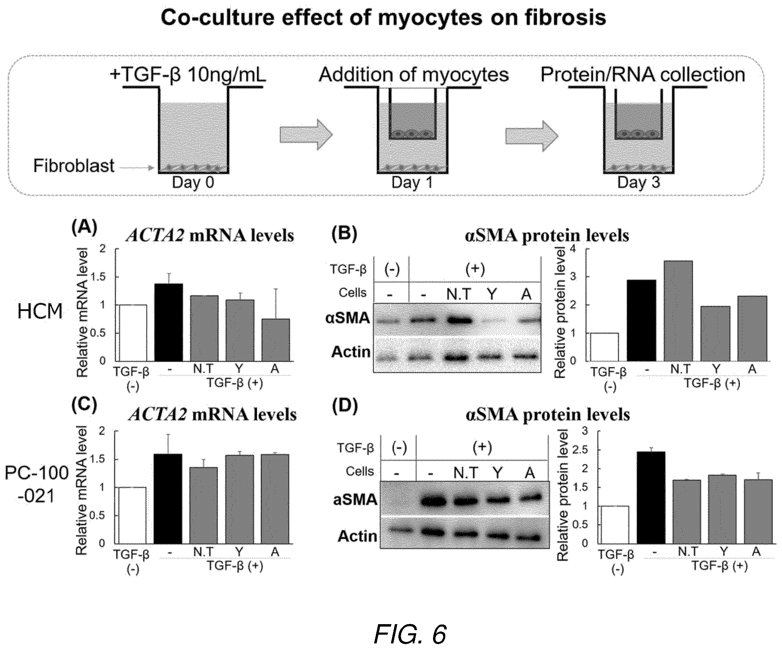

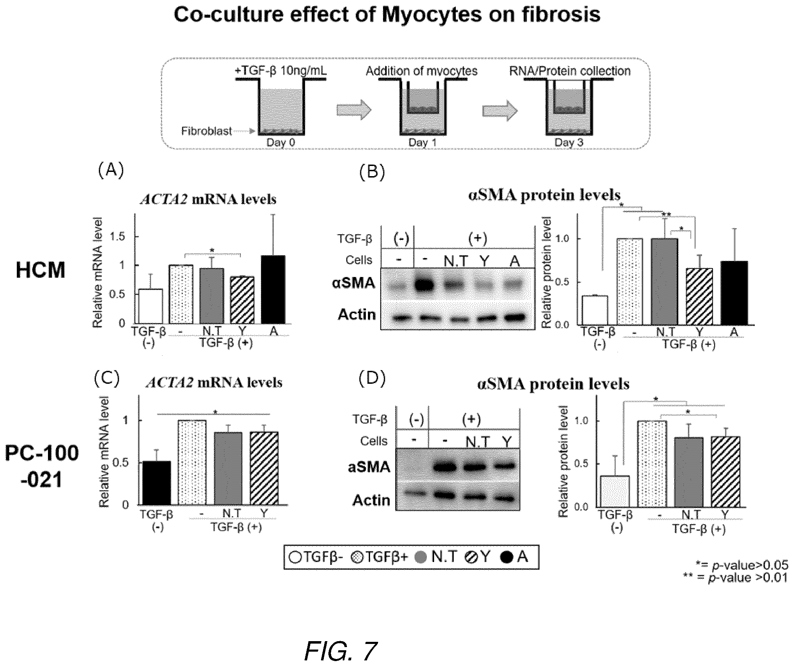

FIG. 6 includes a schematic diagram of an experiment in which cardiomyocytes treated with the TGFβ receptor inhibitor or the ROCK inhibitor and fibroblasts activated by TGFβ treatment were co-cultured, and a diagram showing the results thereof. A and C: Graphs showing ACTA2 mRNA levels of fibroblasts co-cultured with HCM cells (A) or PC-100-021 cells (C). The vertical axis represents an expression amount (Relative mRNA level) of ACTA2 mRNA in each fibroblast when an expression amount of ACTA2 mRNA in fibroblasts not treated with TGFβ is 1. The horizontal axis represents the presence or absence of TGFβ treatment and the agents used for the treatment (in order from the left, TGF-β(-): no TGFβ treatment, no agent treatment, and no co-culture; -: TGFβ treated, agent untreated, and no co-culture; N.T: TGFβ treated, agent untreated, and co-cultured with cardiomyocytes; Y: TGFβ treated, ROCK inhibitor treated, and co-cultured with cardiomyocytes; and A: TGFβ treated, TGFβ receptor inhibitor treated, and co-cultured with cardiomyocytes) (hereinafter, the same applies to the horizontal axis in the graphs of FIGS. 7 to 9). B and D: Photographs and graphs of the results of confirming, by Western blotting, expression levels of αSMA protein in fibroblasts co-cultured with HCM cells (B) or PC-100-021 cells (D) are shown. The photographs indicate, in order from the left, TGF-β(-): no TGFβ treatment, no agent treatment, and no co-culture; -: TGFβ treated, agent untreated, and no co-culture; N.T: TGFβ treated, agent untreated, and co-cultured with cardiomyocytes; Y: TGFβ treated, ROCK inhibitor treated, and co-cultured with cardiomyocytes; and A: TGFβ treated, TGFβ receptor inhibitor treated, and co-cultured with cardiomyocytes (hereinafter, the same applies to the photographs of FIGS. 7 to 9). The graphs represent an expression amount (Relative protein level) of αSMA protein in each fibroblast when an expression amount of αSMA protein in fibroblasts not treated with TGFβ is 1.
FIG. 7 includes a schematic diagram of an experiment in which cardiomyocytes treated with the TGFβ receptor inhibitor or the ROCK inhibitor and fibroblasts activated by TGFβ treatment were co-cultured, and a diagram showing the results thereof. A and C: Graphs showing ACTA2 mRNA levels of fibroblasts co-cultured with HCM cells (A) or PC-100-021 cells (C). The vertical axis represents an expression amount (Relative mRNA level) of ACTA2 mRNA in each fibroblast when an expression amount of ACTA2 mRNA in fibroblasts treated with TGFβ, untreated with the agent, and not co-cultured with cardiomyocytes is 1. B and D: Photographs and graphs of the results of confirming, by Western blotting, expression levels of αSMA protein in fibroblasts co-cultured with HCM cells (B) or PC-100-021 cells (D) are shown. The graphs represent an expression amount (Relative protein level) of αSMA protein in each fibroblast when an expression amount of αSMA protein in fibroblasts treated with TGFβ, untreated with the agent, and not co-cultured with cardiomyocytes is 1.

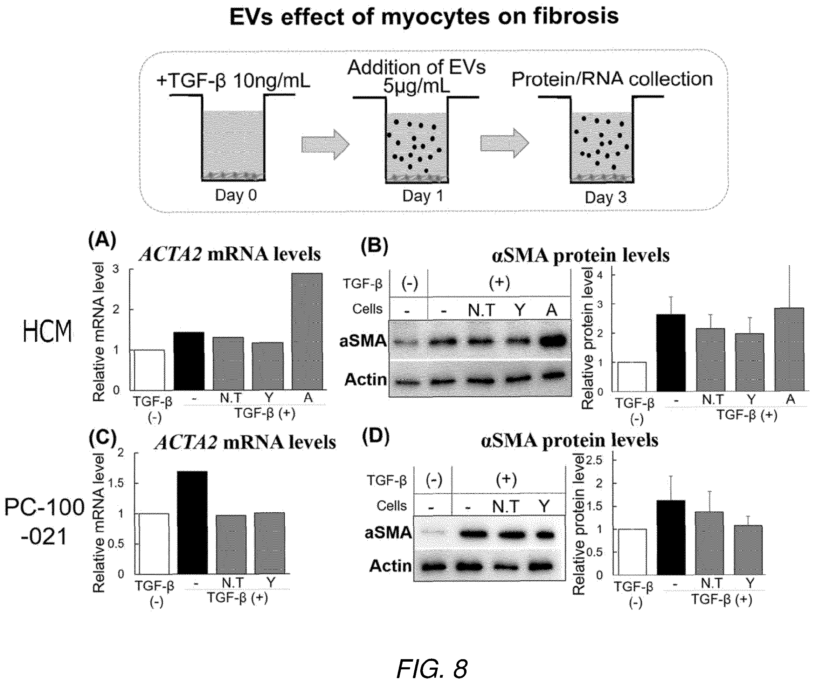

FIG. 8 includes a schematic diagram of an experiment in which exosomes derived from cardiomyocytes treated with the TGFβ receptor inhibitor or the ROCK inhibitor and fibroblasts activated by TGFβ treatment were co-cultured, and a diagram showing the results thereof. A and C: Graphs showing ACTA2 mRNA levels of fibroblasts cultured in the presence of exosomes derived from HCM cells (A) or PC-100-021 cells (C). The vertical axis represents an expression amount (Relative mRNA level) of ACTA2 mRNA in each fibroblast when an expression amount of ACTA2 mRNA in fibroblasts not treated with TGFβ is 1. B and D: Photographs and graphs of the results of confirming, by Western blotting, expression levels of αSMA protein in fibroblasts cultured with exosomes derived from HCM cells (B) or PC-100-021 cells (D) are shown. The graphs represent an expression amount (Relative protein level) of αSMA protein in each fibroblast when an expression amount of αSMA protein in fibroblasts not treated with TGFβ is 1.

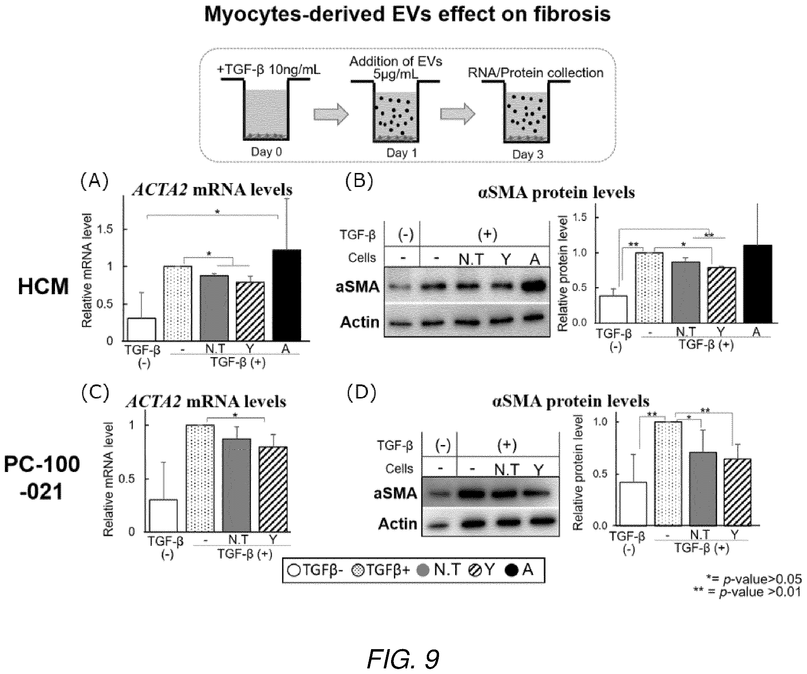

FIG. 9 includes a schematic diagram of an experiment in which exosomes derived from cardiomyocytes treated with the TGFβ receptor inhibitor or the ROCK inhibitor and fibroblasts activated by TGFβ treatment were co-cultured, and a diagram showing the results thereof. A and C: Graphs showing ACTA2 mRNA levels of fibroblasts cultured in the presence of exosomes derived from HCM cells (A) or PC-100-021 cells (C). The vertical axis represents an expression amount (Relative mRNA level) of ACTA2 mRNA in each fibroblast when an expression amount of ACTA2 mRNA in fibroblasts treated with TGFβ, untreated with the agent, and not added with exosomes is 1. B and D: Photographs and graphs of the results of confirming, by Western blotting, expression levels of αSMA protein in fibroblasts cultured with exosomes derived from HCM cells (B) or PC-100-021 cells (D) are shown. The graphs represent an expression amount (Relative protein level) of αSMA protein in each fibroblast when an expression amount of αSMA protein in fibroblasts treated with TGFβ, untreated with the agent, and not added with exosomes is 1.

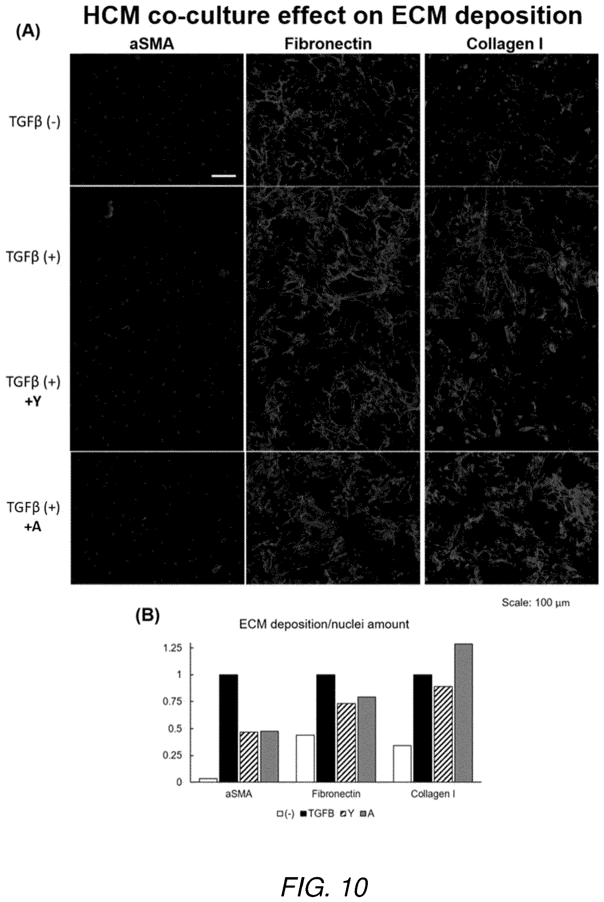

FIG. 10 includes a view (A) and a graph (B) of immunostaining when HCM cells treated with the TGFβ receptor inhibitor or the ROCK inhibitor were co-cultured with fibroblasts activated by TGFβ treatment, and anti-αSMA, anti-fibronectin, and an anti-collagen I antibodies were used as primary antibodies. The view shows, in order from the top, cases of TGFβ untreated and no co-culture (TGFβ(-)), TGFβ treated and co-culture (TGFβ(+)), TGFβ treated and co-culture with ROCK inhibitor-treated cardiomyocytes (TGFβ(+)+Y), and TGFβ treated and co-culture with TGFβ receptor inhibitor-treated cardiomyocytes (TGFβ(+)+A). The graph represents expression amounts of αSMA protein, fibronectin, and collagen I in fibroblasts untreated with TGFβ and not co-cultured (-), TGFβ-treated and co-cultured with ROCK inhibitor-treated cardiomyocytes (Y), or TGFβ-treated and co-cultured with TGFβ receptor inhibitor-treated cardiomyocytes (A), when expression amounts of αSMA protein, fibronectin, and collagen I in fibroblasts treated with TGFβ and non-co-cultured (TGFB) are each 1.

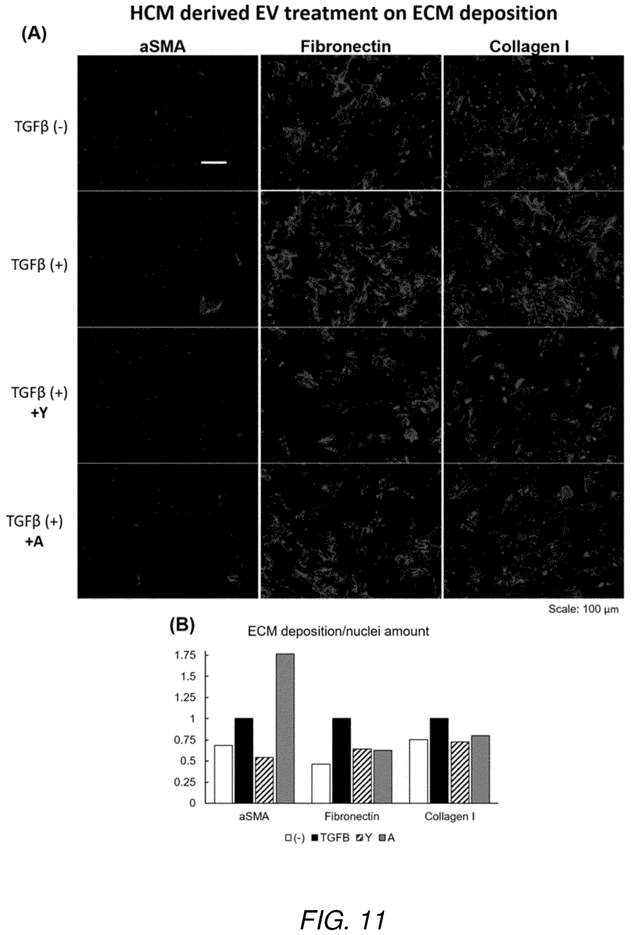

FIG. 11 includes a view (A) and a graph (B) of immunostaining when exosomes derived from HCM cells treated with the TGFβ receptor inhibitor or the ROCK inhibitor were added to fibroblasts activated by TGFβ treatment and they were cultured, and anti-αSMA, anti-fibronectin, and an anti-collagen I antibodies were used as primary antibodies. The view shows, in order from the top, cases of TGFβ untreated and no exosome added (TGF(3(-)), TGFβ treated and no exosome added (TGFβ(+)), TGFβ treated and ROCK inhibitor-treated cardiomyocyte-derived exosomes added (TGFβ(+)+Y), and TGFβ treated and TGFβ receptor inhibitor-treated cardiomyocyte-derived exosomes added (TGFβ(+)+A). The graph represents expression amounts of αSMA protein, fibronectin, and collagen I in fibroblasts untreated with TGFβ and not co-cultured (-), TGFβ-treated and added with ROCK inhibitor-treated cardiomyocyte-derived exosomes (Y), or TGFβ-treated and added with TGFβ receptor inhibitor-treated cardiomyocyte-derived exosomes (A), when expression amounts of αSMA protein, fibronectin, and collagen I in fibroblasts treated with TGFβ (not added with exosomes) (TGFB) are each 1.

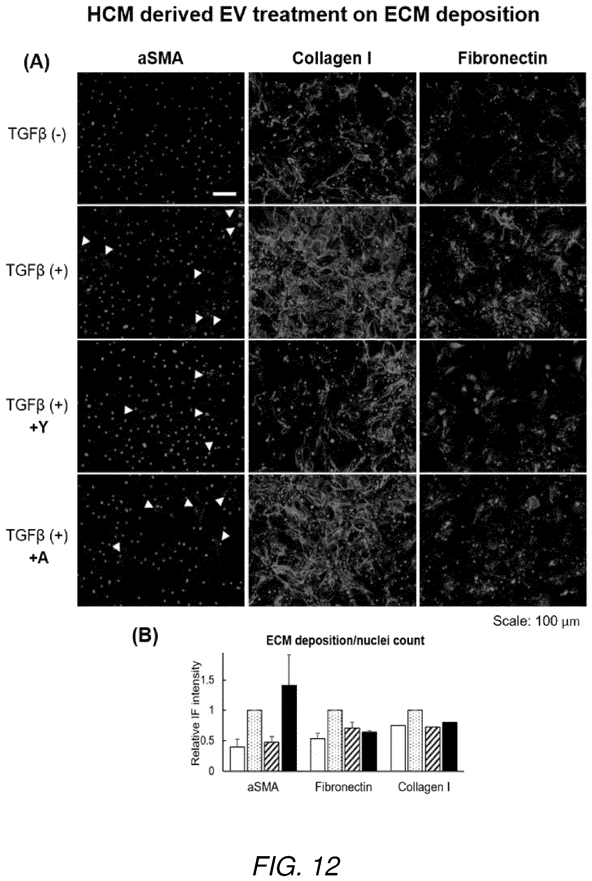

FIG. 12 includes a view (A) and a graph (B) of immunostaining when exosomes derived from HCM cells treated with the TGFβ receptor inhibitor or the ROCK inhibitor were added to fibroblasts activated by TGFβ treatment and they were cultured, and anti-αSMA, anti-fibronectin, and an anti-collagen I antibodies were used as primary antibodies. The view shows, in order from the top, cases of TGFβ untreated and no exosome added (TGF(3(-)), TGFβ treated and no exosome added (TGFβ(+)), TGFβ treated and ROCK inhibitor-treated cardiomyocyte-derived exosomes added (TGFβ(+)+Y), and TGFβ treated and TGFβ receptor inhibitor-treated cardiomyocyte-derived exosomes added (TGFβ(+)+A). αSMA-Positive cells are indicated by white arrows. The graph represents expression amounts of αSMA protein, fibronectin, and collagen I in fibroblasts untreated with TGFβ and not co-cultured, TGFβ-treated and added with ROCK inhibitor-treated cardiomyocyte-derived exosomes, or TGFβ-treated and added with TGFβ receptor inhibitor-treated cardiomyocyte-derived exosomes, when expression amounts of αSMA protein, fibronectin, and collagen I in fibroblasts treated with TGFβ (not added with exosomes) are each 1. The graphs each show, in order from the left, results in fibroblasts untreated with TGFβ and not co-cultured, treated with TGFβ and not added with exosomes, treated with TGFβ and added with ROCK inhibitor-treated cardiomyocyte-derived exosomes, and treated with TGFβ and added with TGFβ receptor inhibitor-treated cardiomyocyte-derived exosomes.

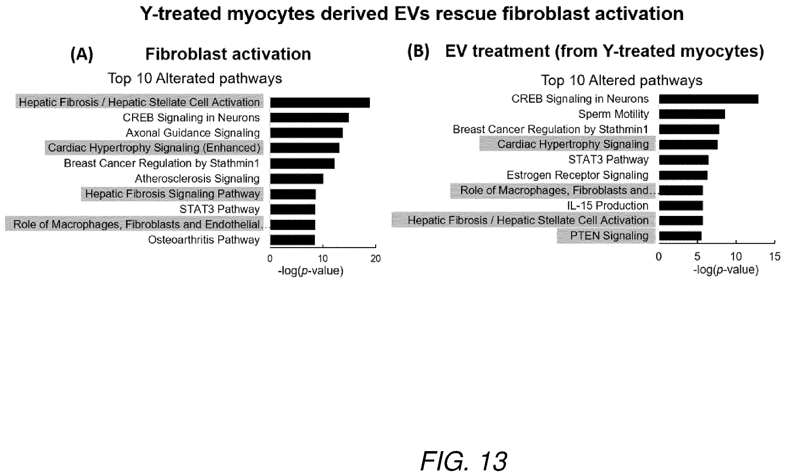

FIG. 13 includes graphs showing results of analyzing changes from existing signaling pathways in fibrotic cells using IPA. The graphs show top 10 signaling pathways changed, (A) when the fibroblasts were subjected to an activation treatment and (B) when the activated fibroblasts were treated with exosomes obtained by treating cardiomyocytes with the ROCK inhibitor.

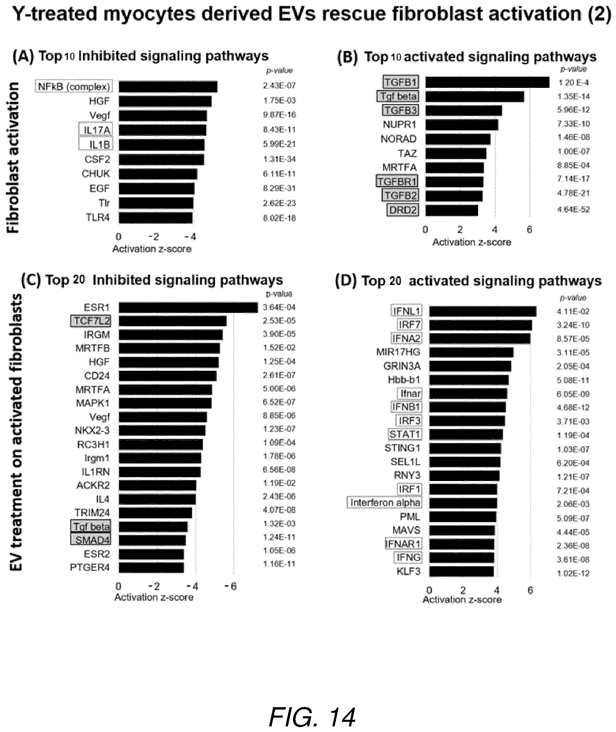

FIG. 14 includes graphs showing results of analyzing (A) top 10 signaling pathways suppressed and (B) top 10 signaling pathways activated, in activation-treated fibroblasts, using IPA. The graphs show results of analyzing (C) top 20 signaling pathways suppressed and (D) top 20 signaling pathways activated, when activated fibroblasts were treated with exosomes obtained by treating cardiomyocytes with the ROCK inhibitor, using IPA.

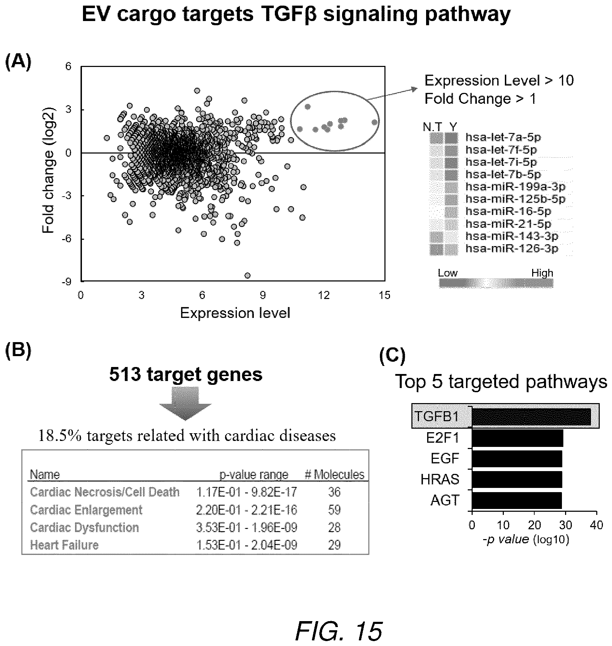

FIG. 15 (A): A graph obtained by analyzing exosomes in which selected microRNA is highly expressed in Example 10. The horizontal axis represents the expression level. (B): A diagram showing that, of 513 genes targeted by the microRNA contained in the selected exosomes, 18.5% are associated with cardiovascular diseases. (C): A diagram showing top 5 signaling pathways involving the genes targeted by the selected microRNA.

### MODE FOR CARRYING OUT THE INVENTION

### 1. Method for producing myocardial stem/progenitor cell from cardiomyocyte, and produced myocardial stem/progenitor cell

In one aspect, the present invention relates to a method for producing myocardial stem/progenitor cells, including treating cardiomyocytes with a ROCK inhibitor (hereinafter, also referred to as "myocardial reprogramming method of the present invention").

The "cardiomyocytes" used in the myocardial reprogramming method of the present invention may be either mature cardiomyocytes or juvenile cardiomyocytes. For example, the cardiomyocytes may be cells expressing at least one cardiomyocyte marker gene (for example, MYL2, cardiac troponin 1, GATA4, VCAM-1, or Nkx2.5), preferably MYL2 and cardiac troponin 1.

An animal from which the cardiomyocytes used in the reprogramming method of the present invention are derived is preferably a mammal, and may be, for example, a human, a rat, a mouse, a guinea pig, a rabbit, a sheep, a horse, a pig, a cow, a monkey, or the like, preferably a human, a rat, or a mouse, and most preferably a human.

The cardiac cells may be cardiomyocytes isolated from a heart extracted from a mammal (primary cardiomyocytes), as well as immortalized cardiomyocytes (for example, cardiomyocytes into which SV40 large T antigen has been introduced), cardiomyocytes obtained from pluripotent stem cells such as embryonic stem cells (ES cells) or iPS cells, or mesenchymal stem cells by a known differentiation inducing method (for example, J. Clin. Inv., 1999; 103: 697-705.; Circ Res. 2009; 104(4): e30-41), cardiomyocytes induced from fibroblasts by direct reprogramming (Circulation Report, Vol. 1 (2019), No. 12: pp. 564-567), and the like, but are preferably primary cardiomyocytes. Alternatively, the cardiomyocytes may be cardiomyocytes present in the heart in the mammalian body.

In the case of using primary cardiomyocytes, for example, in the case of rodents, it is preferable to use a heart extracted from an adult of 10 to 20 weeks of age, but a heart of a juvenile individual of 8 weeks of age or younger may be used. In the case of humans, it is preferable to use an adult heart tissue fragment excised by surgery, but a heart excised from a dead fetus may be used. Alternatively, it is also possible to use cells (frozen cardiomyocytes) obtained by freezing cardiomyocytes isolated and purified from these extracted hearts. As a method for obtaining cardiomyocytes from a mammalian heart or a tissue fragment thereof, a method can be used in which cardiac muscle tissue is digested with collagenase, and non-parenchymal cells and cell fragments are removed by filtration, centrifugation, or the like.

In the present specification, the "myocardial stem/progenitor cells" (hereinafter, also referred to as "CMSCs") means cells having differentiation unipotency and self-renewal ability destined for differentiation into a cardiac muscle. Preferably, the CMSCs herein have a high expression level of a stem cell marker. For example, the expression level of the stem cell marker of the CMSCs herein may be higher as compared to that of mature cardiomyocytes. Herein, the "stem cell marker" means one or more markers selected from Pdx1, Nkx6.1, Gata4, Vcam1, Hes1, Sox9, Foxa2, CK19, and CD133, and is preferably Gata4 and/or Vcam1. Also, preferably, the CMSCs herein have a low expression level of a maturation marker. For example, the expression level of the maturation marker of the CMSCs herein may be low as compared to that of fully differentiated cardiomyocytes. Herein, the "maturation marker" means a maturation marker for cardiomyocytes, and examples thereof include Myl2, Myl7, Myh7, Herg, Kcnq1, Tcap, Vcam1, and Sirpa. In addition, the CMSCs herein have a small number of cell death due to treatment with a ROCK inhibitor. Preferably, the CMSCs herein have a low expression level of a senescence marker. For example, the expression level of the senescence marker of the CMSCs herein may be lower as compared to that of mature cardiomyocytes or ROCK inhibitor-untreated cardiomyocytes. Herein, the "senescence marker" means a senescence marker for cardiomyocytes, and examples thereof include CDKN1A, CDKN2A, p53, and senescence-associated acid β-galactosidase (SA-β-Gal). The levels of the stem cell marker, the maturation marker, the senescence marker, and an endothelial cell marker may be mRNA levels or protein levels.

Herein, the ROCK inhibitor is a substance known to have an action of inhibiting a function of Rho-bound kinase, and examples thereof include N-[3-[2-(4-amino-1,2,5-oxadiazole-3-yl)-1-ethylimidazo[5,4-d]pyridine-6-yl]oxyphenyl]-4-(2-morpholin-4-ylethoxy)benzamide (GSK269962A), fasudil hydrochloride, trans-4-[(1R)-1-aminoethyl]-N-4-pyridinylcyclohexanecarboxamide (Y-27632), and 4-methyl-5-[[(2S)-2 methyl-1,4-diazepan-1-yl]sulfonyl]isoquinoline (H-1152). Y-27632 is preferable. For these ROCK inhibitors, one compound may be used alone, or two or more compounds may be used in combination. The ROCK inhibitor may be in the form of a free body, a salt such as a hydrochloride or a sulfate, a solvate, or a hydrate.

In addition, in the reprogramming method of the present invention, a small molecule signaling pathway inhibitor other than the ROCK inhibitor may be used in combination with the ROCK inhibitor. Examples of the inhibitor include, but are not limited to, a GSK3 inhibitor and a MEK inhibitor.

When the reprogramming method of the present invention is performed *in vitro,* it can be performed by culturing cardiomyocytes in the presence of the inhibitor. Specifically, culture is performed by adding these inhibitors at an effective concentration in a medium. Here, the medium may be any medium that can be used in culture of cardiomyocytes, and examples of commercially available media include CMC media. Alternatively, a medium prepared by adding Supplement Pack Myocyte Cell GM (containing 5% FBS, 5 µg/mL Insulin, 2 ng/mL FGF-b, and 0.5 ng/mL EGF) and 1% Antibiotic x Antimitotic to Myocyte basal medium as described in Examples may be used. A concentration of the ROCK inhibitor added to the medium can be appropriately selected from, for example, ranges of 0.01 to 500 µM, 0.1 to 100 µM, and 1 to 50 µM, and is more preferably 10 µM.

Examples of a culture vessel used in the culture include a dish, a petri dish, a tissue culture dish, a multi-dish, a microplate, a microwell plate, a multi-plate, a multi-well plate, a chamber slide, a Schale dish, a tube, a tray, and a culture bag. As the culture vessel, a culture vessel for performing suspension culture of cells can be used. Alternatively, in the case of a culture vessel for an adhesion site, one whose inner surface is coated with a cell support substrate for the purpose of improving adhesiveness to cells can be used. Examples of such cell support substrates include collagen, atelocollagen, gelatin, matrigel, poly-L-lysine, laminin, and fibronectin.

The cardiomyocytes can be seeded on the culture vessel at a cell density of 10² to 10⁶ cells/cm², preferably 10³ to 10⁵ cells/cm². The cardiomyocytes can be cultured at 30 to 40°C (preferably about 37°C) in a CO₂ incubator (preferably about 5% CO₂ concentration) atmosphere. The culture period is, for example, 1 to 4 weeks, preferably 1 to 3 weeks. Alternatively, the medium may be replaced with a fresh medium (which may contain the inhibitor) every 1 to 3 days. Induction to CMSCs can be confirmed by expression of the stem cell marker in the cultured cardiomyocytes. The obtained CMSCs may be isolated by a method using the stem cell marker (for example, FACS) as necessary.

In the case of passaging, when the cultured cells reach 80% confluence, the cells are trypsinized and dissociated, and seeded on a new culture vessel at a density of 10³ to 10⁵ cells/cm². Preferably, the medium is replaced with a medium containing a ROCK inhibitor. Stable CMSCs can be obtained through about 4 to 6 passages. After 10 passages or more, they may be cloned by a conventional method. As an example of a method for confirming that CMSCs are maintained even after passage, cells having a low density (for example, 10² to 10³ cells/cm²) may be seeded on the culture vessel to continuously observe or measure the form or number of the cells, or expression of a CMSC marker may be confirmed.

*In vivo* treatment of cardiomyocytes with a ROCK inhibitor makes it possible to perform direct reprogramming on cardiomyocytes present in the heart in the living body. In this case, cardiomyocytes in a mammalian heart are treated with a ROCK inhibitor. The ROCK inhibitor may be administered locally to the heart or a site of interest in the heart, or systemically.

The present invention also relates to CMSCs obtained by culturing cardiomyocytes in the presence of a ROCK inhibitor. The CMSCs can be used as a fibrosis suppressor for fibroblasts which will be described below, or can be prepared as cardiomyocytes for transplantation through proliferation and redifferentiation. For example, CMSCs can be redifferentiated into cardiomyocytes by a known differentiation inducing method (for example, J. Clin. Inv., 1999; 103: 697-705.; Circ Res. 2009; 104 (4): e30-41). The CMSCs or cardiomyocytes differentiated from the CMSCs can be used, for example, in evaluation of cardiac toxicity of a test substance, preparation of a cardiac muscle for transplantation, a source from which a secretome to be released is derived, a suppressor for fibrosis of cardiomyocytes, and the like.

A method for evaluating cardiac toxicity of a test substance includes culturing CMSCs or redifferentiated cardiomyocytes in the presence of the test substance. Treatment of the CMSCs or cardiomyocytes with the test substance is usually performed by adding the test substance to a medium or a culture solution for culturing the CMSCs or cardiomyocytes, but is not limited to this method. For example, when the test substance is a protein or the like, the treatment may be performed by introducing a DNA vector expressing the protein into the cells. The method for evaluating cardiac toxicity of a test substance may further include measuring or observing a disorder of CMSCs treated with the test substance, and determining that the test substance has myocardial toxicity when the disorder of the CMSCs is confirmed.

For example, the method for evaluating myocardial toxicity of a test substance may include treating cardiomyocytes with a ROCK inhibitor to obtain CMSCs, treating the obtained CMSCs with a test substance, measuring or observing a disorder of the CMSCs treated with the test substance, and determining that the test substance has myocardial toxicity when the disorder of the CMSCs is confirmed. Alternatively, the method for evaluating myocardial toxicity of a test substance may include treating cardiomyocytes with a ROCK inhibitor to obtain CMSCs, redifferentiating the obtained CMSCs into cardiomyocytes, treating the redifferentiated cardiomyocytes with a test substance, measuring or observing a disorder of the cardiomyocytes treated with the test substance, and determining that the test substance has myocardial toxicity when the disorder of the cardiomyocytes is confirmed. A degree of the disorder may be determined using, for example, a survival rate or form of the CMSCs or cardiomyocytes, or an apoptosis or necrosis marker therefor as an index. Specifically, for example, when the survival rate of CMSCs or cardiomyocytes is reduced by adding a test substance to a culture solution of the CMSCs or cardiomyocytes, the test substance is determined to have cardiac toxicity, and when there is no significant change in survival rate, the test substance is determined not to have cardiac toxicity.

In addition, the CMSCs of the present invention can be used in preparation of a cardiac muscle for transplantation. A method for preparing a cardiac muscle for transplantation may include culturing and proliferating CMSCs, redifferentiating the proliferated CMSCs into cardiomyocytes, and preparing a cardiac muscle for transplantation from the redifferentiated cardiomyocytes. Thus, in one aspect, the present invention relates to a cardiac muscle for transplantation containing the CMSCs of the present invention or cardiomyocytes induced from the CMSCs of the present invention. In addition, the cardiac muscle for transplantation can be used as a therapeutic agent or prophylactic agent for a myocardial disorder. The myocardial disorder refers to a state in which some abnormality occurs in the cardiac muscle and an abnormality occurs in the function of the heart, and includes acute cardiovascular diseases and chronic cardiovascular diseases. Examples of the chronic cardiovascular diseases include cardiomyopathy (dilated cardiomyopathy and the like), myocarditis, myocardial infarction, cardiac hypertrophy, and hypertension. The CMSCs can be used in suspension in an appropriate isotonic buffer (for example, PBS). The CMSC suspension varies depending on the type of heart disease, the severity of myocardial disorder, and the like. For example, in the case of an adult, transplantation can be performed by directly administering 10⁸ to 10¹¹ cells into the cardiac muscle, directly administering the cells from the inside of the atrium to the cardiac muscle with a catheter, or the like. In addition, the cardiac muscle for transplantation may be a cardiac muscle sheet obtained by mixing and culturing cardiomyocytes differentiated from the CMSCs of the present invention with vascular endothelial cells and vascular wall cells. The cardiac muscle sheet is transplanted by being applied to a treatment site of a mammalian heart.

### 2. Method for culturing cardiomyocyte and method for maintenance culture of CMSC

In another aspect, the present invention relates to a method for culturing cardiomyocytes, including culturing cardiomyocytes in the presence of a ROCK inhibitor. In yet another aspect, the present invention relates to a method for maintenance culture of CMSCs, including culturing CMSCs in the presence of a ROCK inhibitor.

The cardiomyocytes and CMSCs can be cultured by subculture according to the culturing method described above. In particular, the culturing method of the present invention makes it possible to culture CMSCs for a long term while maintaining stem cell/progenitor cell properties of the CMSCs. Herein, maintenance of CMSCs or maintenance of stem/progenitor cell properties of CMSCs may mean that a level of the maturation marker is low and/or that a level of the stem cell marker is high. For example, a level of the maturation marker expressed by the cardiomyocytes or CMSCs after an elapse of a long-term culture period may be lower than a level of the maturation marker expressed by the cardiomyocytes cultured in the absence of the ROCK inhibitor. In addition, a level of the stem cell marker expressed by the cardiomyocytes or CMSCs after the elapse of the long-term culture period may be higher than a level of the stem cell marker expressed by the cardiomyocytes cultured in the absence of the ROCK inhibitor. Herein, the "long-term" means that the culture period during which cardiomyocytes or induced CMSCs proliferate is longer than that of cardiomyocytes not treated with the ROCK inhibitor, and may mean 20 days or more, 1 month or more, 40 days or more, or 2 months or more.

### 3. Agent for inducing CMSC from cardiomyocyte, long-term culture agent for cardiomyocyte, or agent for maintaining CMSC

In one aspect, the present invention is an agent for inducing CMSCs from cardiomyocytes, a long-term culture agent for cardiomyocytes, or an agent for maintaining CMSCs, containing a ROCK inhibitor as an active ingredient. The agents containing a ROCK inhibitor may contain a ROCK inhibitor alone as an active ingredient or may contain other agents.

In another aspect, the present invention relates to use of a ROCK inhibitor for the manufacture of an agent for inducing CMSCs from cardiomyocytes. The present invention further relates to a method of inducing CMSCs from cardiomyocytes, including administering, to a patient in need thereof, an effective amount of a ROCK inhibitor. The present invention also relates to a ROCK inhibitor for use in a method for inducing CMSCs from cardiomyocytes.

### 4. Secretome and exosome

In one aspect, the present invention is an exosome or serectome derived from cardiomyocytes cultured in the presence of a ROCK inhibitor. Herein, the "secretome" is a generic term for useful components secreted into cell culture supernatant, and includes, for example, protein components such as various cytokines and chemokines, extracellular matrices such as ECM, and fine particles such as extracellular vesicles. In addition, herein, the "exosome" is a vesicle derived from an endosomal membrane formed in an endocytosis process, which has a diameter of about 20 to 200 nm (preferably 50 to 150 nm) and is released from various cells, and it is composed mainly of lipids, proteins, and nucleic acids (micro RNA, messenger RNA, and DNA).

In another aspect, the present invention relates to a method for preparing a secretome or exosome, including culturing cardiomyocytes in the presence of a ROCK inhibitor, and recovering a secretome or exosome from the cultured cardiomyocytes.

The secretome of the present invention can be obtained as a culture solution (for example, a culture supernatant) in which cardiomyocytes or CMSCs are cultured in the presence of a ROCK inhibitor. Culture of cardiomyocytes and CMSCs can be performed in accordance with the above description.

The exosome of the present invention can be recovered from the secretome. A method for recovering the exosome from the culture solution or secretome can be performed using any known method or a commercially available kit. Examples of the method include ultracentrifugation (for example, Thery C., Curr. Protoc. Cell Biol. (2006) Chapter 3: Unit 3.22.), polymer precipitation, immunoprecipitation, FACS, ultrafiltration, gel filtration, HPLC, and adsorption to a carrier such as beads using an antibody or lectin. In addition, the exosome may be recovered using a commercially available exosome isolation kit.

Among the recovery methods described above, the ultracentrifugation is the standard method most commonly used for exosome isolation. A centrifugal force in the ultracentrifugation may be, for example, 50,000 × g or more, 100,000 × g or more, or 1,500,000 × g or more, and may be 300,000 × g or less, 250,000 × g or less, or 200,000 × g or less. A centrifugation time can be, but is not limited to, for example, 30 minutes to 120 minutes, 60 minutes to 90 minutes, or 70 minutes to 80 minutes. In addition, impurities may be removed or reduced by performing filter filtration and/or centrifugation at a lower centrifugal force as necessary before the centrifugation.

The presence of the exosome can be measured by a nano-particle tracking system (for example, a device such as NanoSight). In addition, on surfaces of particles of the exosome, for example, molecules such as CD9, CD63, and CD81, which are tetraspanins, exist. These molecules can be markers of the exosome. The presence of the exosome can also be confirmed by confirming expression of these proteins and/or genes by an immunological measurement method or the like (for example, Western blot or the like).

### 5. Method for suppressing fibrosis, fibrosis suppressor, defiberizing method, and defiberizing agent

The secretome or exosome released by ROCK inhibitor-treated cardiomyocytes or CMSCs can suppress fibrosis of cardiomyocytes and defiberize fibrotic cardiomyocytes. Therefore, in another aspect, the present invention relates to a fibrosis suppressing method and a defiberizing method using a ROCK inhibitor, cardiomyocytes cultured in the presence of a ROCK inhibitor, or a serectome or exosome released by cardiomyocytes treated with a ROCK inhibitor. In the present method, the ROCK inhibitor may be administered to be caused to directly act on cardiomyocytes in the living body to release the secretome or exosome, cardiomyocytes treated with the ROCK inhibitor may be used, or a serectome or exosome released by cardiomyocytes treated with the ROCK inhibitor may be isolated and/or purified and used. Fibrosis of cardiomyocytes or defiberization of fibrotic cardiomyocytes may be caused by, for example, suppression of a gene included in a signaling pathway involved in activation or fibrosis of fibrotic cells by microRNA contained in a serectome or exosome released by cardiomyocytes treated with a ROCK inhibitor. Examples of such a signaling pathway include TGFB 1, E2F 1, EGF, HRAS, and AGT, and TGFB 1 is preferable.

When a ROCK inhibitor is administered to directly treat cardiomyocytes in the living body, the ROCK inhibitor can act on cardiomyocytes already located in the vicinity of fibroblasts. Therefore, the present invention includes a method for suppressing fibrosis of fibroblasts and a method for defiberizing fibrotic fibroblasts, including treating cardiomyocytes present at a position where a paracrine action can be exerted on fibroblasts with a ROCK inhibitor.

When cells are used, cardiomyocytes treated with a ROCK inhibitor in advance may be used to localize the cardiomyocytes in the vicinity of fibroblasts, or both cells may be co-cultured. Alternatively, cardiomyocytes already located in the vicinity of fibroblasts or co-cultured with fibroblasts may be treated with a ROCK inhibitor. More specifically, the present invention relates to a method for suppressing fibrosis of fibroblasts, including treating cardiomyocytes with a ROCK inhibitor, and localizing the cardiomyocytes with fibroblasts at a position where the cardiomyocytes can exert a paracrine action.

The "paracrine action" means that a substance secreted from ROCK inhibitor-treated cardiomyocytes or CMSCs acts on cells and tissues around the cardiomyocytes or CMSCs. Therefore, the "position where a paracrine action can be exerted" is a position where the serectome or exosome produced from the ROCK inhibitor-treated cardiomyocytes or CMSCs can suppress fibrosis in target fibroblasts, and preferably, the cardiomyocytes or CMSCs are present in the vicinity of or adjacent to the fibroblasts. When cardiomyocytes treated with a ROCK inhibitor in advance are allowed to act on fibroblasts in the living body, the cardiomyocytes can be locally transplanted into the heart or a target site in the heart. That is, transplantation of the inventive ROCK inhibitor-treated cardiomyocytes or CMSCs into the heart makes it possible to release a secretome in a part of the heart and to suppress fibrosis by surrounding fibroblasts by virtue of the paracrine action.

When cardiomyocytes and fibroblasts are co-cultured, they may be cultured on the same surface, or may be cultured in a form in which a serectome or exosome released from the cardiomyocytes can act on the fibroblasts using a chamber or the like. In the case of cultured cells, cardiomyocytes co-cultured with fibroblasts may be treated with a ROCK inhibitor to suppress fibrosis of the fibroblasts.

A culture supernatant of the ROCK inhibitor-treated cardiomyocytes or CMSCs or the exosomes separated and purified from the culture supernatant suppress the fibrosis of the cardiomyocytes. Therefore, the method for suppressing fibrosis of the present invention can also be performed using a secretome or exosome released by ROCK inhibitor-treated cardiomyocytes or CMSCs. Thus, the present invention relates to a method for suppressing fibrosis of fibroblasts, including treating fibroblasts with a secretome or exosome extracted from cardiomyocytes cultured in the presence of a ROCK inhibitor. For example, the method of the present invention may be a method for suppressing fibrosis of fibroblasts including: culturing cardiomyocytes in the presence of a ROCK inhibitor; recovering a secretome or exosome from the cultured cardiomyocytes; and treating fibroblasts with the recovered secretome or exosome.

In the present specification, the method for suppressing fibrosis of fibroblasts may be a method for preventing or treating a disease accompanied by fibrosis of cardiomyocytes, and the fibrosis suppressor for fibroblasts may be a prophylactic or therapeutic agent for a disease accompanied by fibrosis of cardiomyocytes. Examples of the disease accompanied by fibrosis of cardiomyocytes include a disease developed by fibrosis of cardiomyocytes and a disease exacerbated by fibrosis of cardiomyocytes, and include myocardial disorder, cardiac muscle fibrosis, myocardial infarction, heart failure, cardiac hypertrophy, and hypertension.

### 6. Method for suppressing onset and/or exacerbation of cardiovascular disease, and suppressor

The microRNA contained in an exosome derived from cardiomyocytes treated with a ROCK inhibitor targets cardiovascular disease-associated genes, specifically, genes associated with cardiac necrosis and cell death, cardiac enlargement, heart failure, and heart paralysis as the cardiovascular diseases. Therefore, the treatment of cardiomyocytes with a ROCK inhibitor can suppress the onset and/or deterioration of cardiovascular diseases. The treatment of cardiomyocytes with a ROCK inhibitor can also activate signaling pathways related to development and/or functions of the cardiovascular system. This can promote, for example, cell movement, vasculogenesis, angiogenesis, and vasculature development of endothelial cells. Therefore, in another aspect, the present invention relates to a suppressor for onset and/or exacerbation of cardiovascular diseases, a therapeutic agent for a cardiovascular disease, a cell movement promoter for endothelial cells, a vasculogenesis promoter, an angiogenesis promoter, and a vasculature development promoter, each including treating cardiomyocytes with a ROCK inhibitor, a ROCK inhibitor, cardiomyocytes cultured in the presence of a ROCK inhibitor, or a serectome or exosome released by cardiomyocytes treated with a ROCK inhibitor. In the present method, the ROCK inhibitor may be administered to be caused to directly act on cardiomyocytes in the living body to release the secretome or exosome, cardiomyocytes treated with the ROCK inhibitor may be used, or a serectome or exosome released by cardiomyocytes treated with the ROCK inhibitor may be isolated and/or purified and used. In addition, the method may include suppressing a signaling pathway related to the cardiovascular disease and/or activating a signaling pathway related to the development and/or functions of the cardiovascular system by microRNA contained in the exosome derived from the cardiomyocytes treated with the ROCK inhibitor. The cardiovascular disease may be, in addition to the above-described acute cardiovascular diseases and chronic cardiovascular diseases (for example, cardiomyopathy (such as dilated cardiomyopathy), myocarditis, myocardial infarction, cardiac hypertrophy, and hypertension), ventricular dysfunction, left ventricular dysfunction, left heart disease, dysfunction of heart, familial cardiovascular disease, cerebrovascular dysfunction, abnormality of left ventricular, abnormality of heart ventricle, peripheral vascular disease, atherosclerosis, arteriosclerosis, occlusion of blood vessel, vaso-occlusion, occlusion of artery, congestive heart failure, and heart failure.

In another aspect, the present invention relates to a method for suppressing or treating the onset and/or exacerbation of a cardiovascular disease, including administering an effective amount of a ROCK inhibitor, cardiomyocytes treated with a ROCK inhibitor, or a secretome or exosome released by cardiomyocytes treated with a ROCK inhibitor to a patient in need thereof. In addition, the present invention relates to use of a ROCK inhibitor, cardiomyocytes treated with a ROCK inhibitor, or a secretome or exosome released by cardiomyocytes treated with a ROCK inhibitor, for the manufacture of a suppressor or therapeutic agent for onset and/or exacerbation of a cardiovascular disease. In addition, the present invention relates to a ROCK inhibitor, cardiomyocytes treated with a ROCK inhibitor, or a secretome or exosome released by cardiomyocytes treated with a ROCK inhibitor, for suppressing or treating onset and/or exacerbation of a cardiovascular disease.

### 7. Administration method and formulation

The serectome and exosome can be obtained from cardiomyocytes treated with a ROCK inhibitor by the above-described method. When treatment with the serectome or exosome is performed *in vitro,* the treatment can be performed by culturing cardiomyocytes or fibroblasts in the presence of the serectome or exosome. When the treatment is performed *in vivo,* the serectome or exosome may be locally administered to the heart or a target site in the heart, or may be systemically administered.

The above-described agents may contain, as an active ingredient, a ROCK inhibitor, cardiomyocytes treated with a ROCK inhibitor, or a secretome or exosome released by cardiomyocytes treated with a ROCK inhibitor alone, or may contain other components as necessary. For example, when the agent is intended for administration to an animal, the other components may be a pharmaceutically acceptable additive, for example, sterile water, physiological saline, a buffer, an excipient, a binder, a disintegrant, an emulsifier, a surfactant, a stabilizer, a lubricant, a diluent, a flowability accelerator, a flavoring agent, a coloring agent, a fragrance, and the like. For example, when the agent of the present invention is intended for administration to an animal, the agent can be administered in an oral administration form or a parenteral administration form such as an injection or a drip. In addition, these agents may be orally administered as tablets, powders, granules, syrups, or the like, or may be parenterally administered as injections or drips. Any dose may be employed as long as it is effective for achieving the purpose, and can be determined according to symptom, age, sex, body weight, administration form, and the like.

The methods of the present invention can all be performed *in vivo, ex vivo,* or *in vitro,* except where such interpretation is inconsistent.

Hereinafter, the invention will be described in more detail using examples, which are not intended to limit the scope of the invention. It should be noted that the documents cited throughout the present specification are incorporated herein by reference in their entirety.

### EXAMPLES

### (Preparation of medium)

A cardiomyocyte medium (CMM) was adjusted as follows: Supplement Pack Myocyte Cell GM (containing 5% FBS, 5 µg/mL Insulin, 2 ng/mL FGF-b, and 0.5 ng/mL EGF) (PromoCell, Cat # C-39270) and 1% Antibiotic x Antimitotic were added to Myocyte basal medium.

A culture medium for PC-100-021 was adjusted as follows: Vascular Smooth Muscle Cell growth Kit (containing 5% FBS, 5% L-Glutamine, 50 µg/mL Ascorbic acid, 5 ng/mL EGF, 5 µg/mL Insulin, and 5 ng/mL FGF-b) (ATCC, Cat # PCS-100-042) and 1% Antibiotic × Antimitotic were added to Vascular Smooth Muscle Cell growth medium.

Advanced DMEM (Gibco, cat. No. 12491) was used as a complete medium.

### (Example 1) Culture of cardiomyocyte

### (1) Primary culture

Primary human cardiomyocytes (HCM, PromoCell) and primary human coronary artery smooth muscle cells (PC-100-021, ATCC) as a control were seeded on a culture dish containing a cardiomyocyte medium (CMM) suitable for culturing cardiomyocytes and a culture medium for PC-100-021, respectively, and plate-cultured in an incubator (37°C, 5% CO₂/95% air).

### (2) Subculture

The human cardiomyocytes in the primary culture described above were treated with TrypLE Express (ThermoFisher) and recovered, and seeded at 9 × 10³ cells/cm² in a culture vessel coated with collagen I (2 µg/cm², 150 mm dish), and cultured in Vascular Smooth Muscle Cell growth medium or Myocyte basal medium. A frozen stock of the cultured cells was prepared using CELLBANKER (registered trademark) 1 (Takara Bio Inc.).

### (Example 2) Action of TGFβ receptor inhibitor and ROCK inhibitor in cardiomyocyte culture

### (1) Culture in presence of test low molecular weight compound

The subcultured human cardiomyocytes were seeded at 0.5×10² cells/cm² on a 35 mm plate (IWAKI) containing: 3 mL of a Vascular Smooth Muscle Cell growth medium containing 3-(6-methyl-2-pyridinyl)-n-phenyl-4-(4-quinolinyl)-1H-pyrazole-1-carbothioamide (A-83-01: A) (final concentration: 1 µM) as a low molecular weight compound TGFβ receptor inhibitor or (1R,4r)-4-((R)-1-aminoethyl)-N-(pyridin-4-yl)cyclohexanecarboxamide (Y-27632: Y) (final concentration: 10 µM) as a ROCK inhibitor, both of the compounds A-83-01 and Y-27632, or free of them; or a Myocyte basal medium. On Day 3, the Vascular Smooth Muscle Cell growth medium containing each low molecular weight compound or the Myocyte basal medium was replaced. Thereafter, the medium was replaced in the same manner every three days.

### (2) Low-speed imaging at low cell density

After the medium replacement, low-speed imaging was performed using a BZ9000 all-in-one fluorescence microscope (KEYENCE). In addition, the individual cells were tracked throughout the imaging period (110 days), and the final number of cells derived from each cell was measured.

FIG. 1A shows changes in cell morphology of cardiomyocytes treated with A-83-01 or Y-27632. By the treatment with A-83-01 or Y-27632, the cardiomyocytes exhibited a myocardial stem/progenitor cell-like form. In HCM, long-term culture was induced by the A-83-01 treatment and Y-27632 treatment (FIG. 1B). In PC-100-021, long-term culture was induced by the A-83-01 treatment (FIG. 1C).

### (3) Quantitative RT-PCR

Total RNA was isolated from each cardiomyocyte 1 month and 2 months after culture in the presence of the test low molecular weight compound using miRNeasy Mini Kit (QIAGEN). Reverse transcription reactions were performed using High-Capacity cDNA Reverse Transcription Kit Lifetechnologies according to manufacturer's guidelines. PCR was performed using the obtained cDNA as a template and a Taqman Probe (ThermoFisher). An expression level of a target gene was standardized with β-actin as an endogenous control.

FIG. 2 shows expression amounts of mRNA of myocardial progenitor cell markers (GATA4 and VCAM-1) and mRNA of MYL2 as a maturation marker for cardiomyocytes after culturing HCM cells and PC-100-021 cells in the presence of A-83-01 (A) or Y-27632 (Y). For both of the HCM cells and the PC-100-021 cells, in the agent untreated group (N.T.), differentiation proceeded by continuing the culture, and the expression of the progenitor cell markers (GATA4 and VCAM-1) decreased and the expression of the maturation marker (MYL2) increased. This decrease in progenitor cell markers (GATA4 and VCAM-1) and increase in maturation marker (MYL2) were suppressed by treating the HCM cells and the PC-100-021 cells with Y-27632. On the other hand, it was shown that the treatment with A-83-01 promoted the decrease in progenitor cell markers (GATA4 and VCAM-1) and an increase in maturation marker (MYL2).

FIG. 3A shows expression amounts of mRNA of the myocardial progenitor cell markers (GATA4 and VCAM-1), mRNA of MYL2 as the maturation marker for cardiomyocytes and mRNA of senescence markers (CDKN1A and CDKN2A) for cardiomyocytes after culturing the HCM cells and the PC-100-021 cells in the presence of A-83-01 (A) or Y-27632 (Y). For both of the HCM cells and the PC-100-021 cells, in the agent untreated group (N.T.), differentiation proceeded by continuing the culture, and the expression of the progenitor cell markers (GATA4 and VCAM-1) decreased and the expression of the maturation marker (MYL2) increased. This decrease in progenitor cell markers (GATA4 and VCAM-1) and increase in maturation marker (MYL2) were suppressed by treating the HCM cells and the PC-100-021 cells with Y-27632. On the other hand, the treatment with A-83-01 was shown to promote the increase in maturation marker (MYL2). Furthermore, the senescence markers (CDKN1A and CDKN2A) were hardly expressed in the HCM cells and PC-100-021 cells treated with A-83-01(A) or Y-27632(Y). Therefore, it was shown that the number of cell death due to the treatment with A-83-01(A) or Y-27632(Y) was small. In addition, from FIG. 3B, it was confirmed that, in the HCM cells and PC-100-021 cells treated with A-83-01 (A) or Y-27632 (Y), the endothelial cell marker (CD31) was not expressed and the muscle cell marker was expressed; that these cells contained only muscle cells; and that endothelial cells were not mixed in the primary culture.

### (Example 3) Property regarding signaling pathway of cardiomyocyte treated with ROCK inhibitor

Signaling pathways that would be changed when cardiomyocytes were treated with Y-27632 (Y) were estimated, as compared to those in the case of untreated cardiomyocytes. For the estimation, Ingenuity (registered trademark) Pathway Analysis (IPA) (QIAGEN) was used according to the manufacturer's instruction manual.

In cardiomyocytes treated with the ROCK inhibitor Y-27632 (Y), pathways associated with circulatory diseases were inhibited, as compared to untreated cardiomyocytes. Specifically, it was estimated that signaling pathways associated with ventricular dysfunction, left ventricular dysfunction, left heart disease, dysfunction of heart, familial cardiovascular disease, cerebrovascular dysfunction, abnormality of left ventricular, abnormality of heart ventricle, peripheral vascular disease, atherosclerosis, arteriosclerosis, occlusion of blood vessel, vaso-occlusion, occlusion of artery, congestive heart failure, and heart failure would be suppressed (FIG. 4A). Thus, it was suggested that signaling pathways associated with cardiovascular diseases are reduced in the ROCK inhibitor-treated cardiomyocytes. In addition, it was estimated that the treatment of cardiomyocytes with Y-27632 (Y) would suppress signaling pathways associated with cardiac contraction, blood pressure, and myocardial contraction, and activate signaling pathways associated with cell movement, vasculogenesis, angiogenesis, and vasculature development of endothelial cells (FIG. 4B). Therefore, it was suggested that, in the ROCK inhibitor-treated cardiomyocytes, signaling pathways related to the development and functions of the cardiovascular system would be activated.

In addition, from such a change in the signal system, it was suggested that the ROCK inhibitor-treated cardiomyocytes exhibit regenerative properties and would be less likely to undergo malignant transformation.

### (Example 4) Purification and analysis of exosome from myocardial stem/progenitor cell

Until myocardial stem/progenitor cells reached 70% confluence, cells were cultured in a complete medium. As the complete medium, the above-described medium was used. Thereafter, the complete medium was replaced with Advanced DMEM (Gibco) and culture was further performed for 48 hours. Thereafter, the culture solution was recovered, centrifuged (2,000 × g, 10 min, 4°C), and filtered through a 0.22 µm filter. Then, a cell pellet was discarded, and a supernatant was recovered. Exosomes were isolated from the supernatant continuously by ultracentrifugation. Then, the exosome were ultracentrifuged (35,000 × g, 70 min, 4°C), dissolved in PBS, and stored at 4°C.

FIG. 5 shows the number of particles obtained by measuring the exosome (EV), which is a type of secretome purified by ultracentrifugation from the culture supernatant of the cardiomyocytes treated with A-83-01 and Y-27632, with a nanoparticle trapping system (Nanosight LM-10) (A). Furthermore, the presence of a CD9 molecule, a CD63 molecule, and a CD81 molecule contained in the exosome identified by Western blotting is shown. From this, it was shown that the exosome could actually be recovered.

### (Example 5) Marker expression in TGFβ-stimulated fibroblast co-cultured with cardiomyocyte treated with TGFβ receptor inhibitor and ROCK inhibitor

Human cardiac fibroblasts were activated with TGFβ for 24 hours. The activated fibroblasts were co-cultured with A-83-01- or Y-27632-pretreated cardiomyocytes for 48 hours or more. Total RNA and protein were extracted for fibrosis activation marker analysis.

FIG. 6 includes graphs showing mRNA levels of a fiber-relevant gene ACTA2 (A and C) and protein levels of a fibrosis marker αSMA (B and D) in TGFβ-stimulated fibroblasts co-cultured with cardiomyocytes treated with A-83-01 and Y-27632. The expression levels of ACTA2 and αSMA can be indicative of cellular fibrosis. The ACTA2 and αSMA expression levels increased with fibrosis of human cardiac fibroblasts by TGFβ treatment, but decreased by co-culture with cardiomyocytes, and, from the fact, it was seen that fibroblast activation was suppressed. Therefore, an effect of suppressing activation of fibroblasts by the secretome purified from the cardiomyocytes treated with the TGFβ receptor inhibitor and the ROCK inhibitor was shown.

FIG. 7 includes graphs showing mRNA levels of the fiber-relevant gene ACTA2 (A and C) and protein levels of the fibrosis marker αSMA (B and D) in the TGFβ-stimulated fibroblasts co-cultured with the cardiomyocytes treated with A-83-01 and Y-27632. The graphs are shown as mean ± standard deviation. The expression levels of ACTA2 and αSMA can be indicative of cellular fibrosis. The ACTA2 and αSMA expression levels increased with fibrosis of human cardiac fibroblasts by TGFβ treatment, but decreased by co-culture with cardiomyocytes, and, from the fact, it was seen that fibroblast activation was suppressed. Therefore, an effect of suppressing activation of fibroblasts by secretome purified from the cardiomyocytes treated with the TGFβ receptor inhibitor and the ROCK inhibitor was shown.

### (Example 6) Marker expression in TGFβ-stimulated fibroblast cultured in presence of exosome derived from cardiomyocyte treated with TGFβ receptor inhibitor and ROCK inhibitor

Human cardiac fibroblasts were activated with TGFβ for 24 hours. Next, exosomes (EVs) derived from Y-27632-treated cardiomyocytes were added, and fibroblasts were cultured for further 48 hours. Total RNA and protein were extracted for fibrosis activation marker analysis.

FIG. 8 includes graphs showing mRNA levels of ACTA2 (A and C) and protein levels of αSMA (B and D), expressed by fibroblasts in which exosomes purified from cardiomyocytes treated with A-83-01 and Y-27632 were taken in fibroblasts activated by TGFβ treatment. The ACTA2 and αSMA expression levels increased with fibrosis of human cardiac fibroblasts by TGFβ treatment, but significantly decreased by the addition of exosomes, and, from the fact, it was seen that fibroblast activation was suppressed. Therefore, an effect of suppressing activation of fibroblasts by exosomes purified from the cardiomyocytes treated with A-83-01 and Y-27632 was shown.

FIG. 9 includes graphs showing mRNA levels of ACTA2 (A and C) and protein levels of αSMA (B and D), expressed by fibroblasts in which exosomes purified from cardiomyocytes treated with A-83-01 and Y-27632 were taken in fibroblasts activated by TGFβ treatment. The graphs are shown as mean ± standard deviation. The ACTA2 and αSMA expression levels increased with fibrosis of human cardiac fibroblasts by TGFβ treatment, but significantly decreased by the addition of exosomes, and, from the fact, it was seen that fibroblast activation was suppressed. Therefore, an effect of suppressing the activation of fibroblasts by exosomes purified from the cardiomyocytes treated with A-83-01 and Y-27632 was shown.

### (Example 7) Immunostaining

As in Example 5 or 6, fibroblasts activated with TGFβ for 24 hours were co-cultured with HCM cells treated with A-83-01 or Y-27632, or cultured for 48 hours in the presence of exosomes (EVs) derived from the HCM cells. Next, the cells were washed twice with PBS, and then 4% paraformaldehyde was added thereto for fixation for 10 minutes. Cell membranes were permeabilized with 0.1% Triton X dissolved in PBS. Blocking was performed in Blocking One (Nacalai Tesque) for 30 minutes, and the cells were incubated with primary antibodies (anti-αSMA, anti-fibronectin, and anti-collagen I) for 1 hour at room temperature. Secondary antibodies bound to AlexaFluor 594 were incubated further for 1 hour. Nuclei were stained with DAPI (Vectashield).

The results of immunostaining when HCM treated with A-83-01 or Y-27632 and fibroblasts activated by TGFβ treatment were co-cultured are shown in FIG. 10. It was found that the expression of αSMA, fibronectin, and collagen I was significantly suppressed by co-culture with HCM treated with the ROCK inhibitor, and an effect of suppressing fibrosis of cardiac fibroblasts could be confirmed.

The results of immunostaining when exosomes derived from HCM treated with A-83-01 or Y-27632 were added to fibroblasts activated by TGFβ treatment, and they were co-cultured are shown in FIG. 11. It was found that the expression of αSMA, fibronectin, and collagen I was significantly suppressed by the exosomes derived from HCM treated with the ROCK inhibitor, and an effect of suppressing fibrosis of cardiac fibroblasts could be confirmed.

The typical results of immunostaining when exosomes derived from HCM treated with A-83-01 or Y-27632 were added to fibroblasts activated by TGFβ treatment, and they were co-cultured are shown in FIG. 12. αSMA-Positive cells are indicated by white arrows. The graph is shown as mean ± standard deviation. It was found that the TGFβ treatment induced the expression of αSMA and increased the number of αSMA-positive cells, but that the addition of the exosomes derived from HCM treated with Y-27632 suppressed the expression by TGFβ and decreased the number of αSMA positive-cells. Similarly, it was found that the expression of fibronectin and collagen I was also significantly suppressed by the exosomes derived from HCM treated with Y-27632, and an effect of suppressing fibrosis of cardiac fibroblasts could be confirmed.

### (Example 8) Change in signaling pathway in fibroblast cultured in presence of exosome derived from cardiomyocyte treated with ROCK inhibitor

In activated fibroblasts and fibroblasts treated with exosomes derived from cardiomyocytes treated with Y-27632 (Y), signaling pathways which would be changed were estimated, as compared to untreated cardiomyocytes. For the estimation, IPA was used according to the manufacturer's instruction manual.

It was estimated that, in the activated fibroblasts, the associated signaling pathways would be promoted in the order of hepatic fibrosis/hepatic stellate cell activation, CREB signaling in neurons, axonal guidance signaling, cardiac hypertrophy signaling (enhanced), breast cancer regulation by Stathmin1, atherosclerosis signaling, hepatic fibrosis signaling pathway, STAT3 pathway, role of macrophages, fibroblasts, and endothelial, and osteoarthritis pathway (FIG. 13A). On the other hand, it was estimated that, in the fibroblasts treated with exosomes derived from cardiomyocytes treated with Y-27632 (Y), the associated signaling pathways would be promoted in the order of CREB signaling in neurons, sperm motility, breast cancer regulation by Stathmin1, cardiac hypertrophy signaling, STAT3 pathway, estrogen receptor signaling, role of macrophages, fibroblasts, and endothelium, IL-15 production, hepatic fibrosis/hepatic stellate cell activation, and PTEN signaling (FIG. 13B).

### (Example 9) Change in activation signaling factor in fibroblast cultured in presence of exosome derived from cardiomyocyte treated with ROCK inhibitor

In order to estimate more specific signaling factors for the signaling pathways estimated in Example 8, a signaling pathway suppressed or promoted in activated fibroblasts as compared to untreated cardiomyocytes, and a signaling pathway suppressed or promoted when activated fibroblasts were treated with exosomes derived from HCM cells treated with a ROCK inhibitor as compared to untreated cardiomyocytes when were estimated. For the estimation, IPA was used according to the manufacturer's instruction manual.

It was estimated that, in activated fibroblasts, the associated signaling pathways would be suppressed in the order of NFκB (complex), HGF, Vegf, IL17A, IL1B, CSF2, CHUK, EGF, Tlr, and TLR4, and that the associated signaling pathways would be promoted in the order of TGFB1, Tgf beta, TGFB3, NUPR1, NORAD, TAZ, MRTFA, TGFBR1, TGFB2, and DRD2 (FIGS. 14A and 14B).

On the other hand, it was estimated that, when activated fibroblasts were treated with exosomes derived from HCM cells treated with a ROCK inhibitor, the associated signaling pathways would be suppressed in the order of ESR1, TCF7L2, IRGM, MRTFB, HGF, CD24, MRTFA, MAPK1, Vegf, NKX2-3, RC3H1, Irgm1, IL1RN, ACKR2, IL4, TRIM24, Tgf beta, SMAD4, ESR2, and PTGER4, and that the associated signaling pathways would be promoted in the order of IFNL1, IRF7, IFNA2, MIR17HG, GRIN3A, Hbb-b1, Ifnar, IFNB1, IRF3, STAT1, STING1, SEL1L, RNY3, IRF1, Interferon alpha, PML, MAVS, IFNAR1, IFNG, and KLF3 (FIGS. 14C and 14D). Therefore, it was found that the signaling pathways activated in activated fibroblasts were suppressed by the treatment of exosomes. Therefore, it was suggested that specific fibroblast signaling pathways were restored by the treatment of exosomes derived from cardiomyocytes treated with a ROCK inhibitor.

### (Example 10) Functional analysis of microRNA

For exosomes derived from HCM cells treated with a ROCK inhibitor, an exosome group in which microRNA contained therein was most highly expressed was selected (see FIG. 15A), and a target gene of the microRNA was identified. Specifically, the culture solution of HCM cells treated with a ROCK inhibitor was replaced with a serum-free culture solution, and, after culturing for 72 hours, 500 ml of the culture supernatant was recovered. The culture supernatant was centrifuged at 10,000 × g for 30 minutes to remove cell debris and the like, and then ultracentrifuged with an ultracentrifuge manufactured by Beckman Coulter, Inc. (Optima-XE-90) at 100,000 × g and 4°C for 70 minutes. Then, the pellet was dissolved in PBS (-), further ultracentrifuged at 100,000 × g, 4°C for 70 minutes, and then dissolved in an appropriate amount of PBS (-). From this exosome fraction, microRNA was recovered using a micro RNAeasy kit (QIAGEN), and 8 ng of the microRNA was subjected to RNA analysis by a next generation sequencer (DNA Chip Research Inc.).

The results showed that 18.5% of 513 genes targeted by the selected microRNA were associated with cardiovascular diseases such as cardiac necrosis/cell death, cardiac enlargement, and heart failure (see FIG. 15B). In addition, it was shown that the target gene of the selected microRNA was deeply involved in TGFB1 signaling pathway (see FIG. 15C).

## Claims

1. A method for producing myocardial stem/progenitor cells, comprising treating cardiomyocytes with a ROCK inhibitor.

2. A method for culturing cardiomyocytes, comprising culturing cardiomyocytes in the presence of a ROCK inhibitor.

3. The culturing method according to claim 2, wherein a culture period is one month or longer, and/or a level of one or more markers selected from a maturation marker, a senescence marker, and an endothelial cell marker for cardiomyocytes, expressed by the cardiomyocytes after an elapse of the culture period, is lower than a level of the marker for cardiomyocytes expressed by cardiomyocytes cultured in the absence of the ROCK inhibitor.

4. The culturing method according to claim 3, wherein the maturation marker is MYL2, and/or the senescence marker is CDKN1A and/or CDKN2A, and/or the endothelial cell marker is CD31.

5. The culturing method according to claim 3 or 4, wherein a level of a myocardial progenitor cell marker expressed by the cardiomyocytes after the elapse of the culture period is higher than a level of the marker for cardiomyocytes expressed by the cardiomyocytes cultured in the absence of the ROCK inhibitor.

6. A method for maintenance culture of myocardial stem/progenitor cells, comprising culturing myocardial stem/progenitor cells in the presence of a ROCK inhibitor.

7. A method for preparing a secretome or exosome, comprising recovering a secretome or exosome from cardiomyocytes cultured by the method according to any one of claims 2 to 5.

8. A method for suppressing fibrosis of fibroblasts, comprising localizing cardiomyocytes treated with a ROCK inhibitor with fibroblasts at a position where the cardiomyocytes can exert a paracrine effect.

9. A method for suppressing fibrosis of fibroblasts, comprising treating, with a ROCK inhibitor, cardiomyocytes present at a position where a paracrine effect can be exerted on fibroblasts.

10. A method for suppressing fibrosis of fibroblasts, comprising treating fibroblasts with a secretome or exosome extracted from cardiomyocytes cultured in the presence of a ROCK inhibitor.

11. The method for suppressing fibrosis of fibroblasts according to claim 8, comprising:
culturing cardiomyocytes in the presence of the ROCK inhibitor;
recovering a secretome or exosome from the cultured cardiomyocytes; and
treating the fibroblasts with the recovered secretome or exosome.

12. The method according to any one of claims 1 to 11, wherein the ROCK inhibitor is (1R,4r)-4-((R)-1-aminoethyl)-N-(pyridin-4-yl)cyclohexanecarboxamide.

13. The method according to any one of claims 1 to 12, wherein the cardiomyocytes are human cardiomyocytes.

14. An inducer for myocardial stem/progenitor cells or a maintenance culture agent for myocardial stem/progenitor cells, comprising a ROCK inhibitor as an active ingredient.

15. Myocardial stem/progenitor cells obtained by culturing cardiomyocytes in the presence of a ROCK inhibitor.

16. An exosome or serectome derived from cardiomyocytes cultured in the presence of a ROCK inhibitor.

17. The exosome or serectome according to claim 16, which comprises microRNA targeting a cardiovascular disease-associated gene.

18. The exosome or serectome according to claim 17, wherein the cardiovascular disease-associated gene is a gene involved in a TGFB1 signaling pathway.

19. A fibrosis suppressor or defiberizing agent, comprising a ROCK inhibitor, cardiomyocytes cultured in the presence of a ROCK inhibitor, or the exosome or serectome according to any one of claims 16 to 18 as an active ingredient.

20. The inducer for myocardial stem/progenitor cells or the maintenance culture agent for myocardial stem/progenitor cells according to claim 14, the myocardial stem/progenitor cells according to claim 15, the exosome or serectome according to any one of claims 16 to 18, or the fibrosis suppressor or defiberizing agent according to claim 19, wherein the ROCK inhibitor is (1R,4r)-4-((R)-1-aminoethyl)-N-(pyridin-4-yl)cyclohexanecarboxamide.

21. A suppressor or therapeutic agent for onset and/or exacerbation of a cardiovascular disease, comprising a ROCK inhibitor for acting on cardiomyocytes as an active ingredient.

22. The suppressor or therapeutic agent according to claim 21, wherein the cardiovascular disease is at least one disease selected from the group consisting of ventricular dysfunction, left ventricular dysfunction, left heart disease, dysfunction of heart, familial cardiovascular disease, cerebrovascular dysfunction, abnormality of left ventricular, abnormality of heart ventricle, peripheral vascular disease, atherosclerosis, arteriosclerosis, occlusion of blood vessel, vaso-occlusion, occlusion of artery, congestive heart failure, and heart failure.

23. An agent for promoting cell movement, vasculogenesis, angiogenesis, and vasculature formation of endothelial cells, comprising a ROCK inhibitor for acting on cardiomyocytes as an active ingredient.
